# EUROPEAN PATENT APPLICATION

(11) **EP 4 851 202 A1**
(43) Date of publication of application: **09.12.2026**
(21) Application number: 26182302.5
(22) Date of filing: 01.06.2026
(51) Int. Cl.: C08F 6/00, A61L 15/22, C08L 33/06, C08L 33/24

(54) **HYDROGEL COMPOSITION AND HYDROGEL FORMED BY USING THE SAME**

(30) Priority: 02.06.2025 KR 20250072265
(71) Applicant: Aldaver Inc., Daejeon 34051 (KR)
(72) Inventor: KIM, Jin-Oh, 34051 Daejeon (KR); KIM, Ikjin, 34051 Daejeon (KR); RYU, Gunyoung, 34051 Daejeon (KR)
(74) Representative: dompatent

(57) **Abstract**

The present invention relates to a hydrogel composition, comprising: an acrylic compound including acrylamide, methacrylamide, acrylic acid, or a combination thereof; polyethylene glycol having a weight average molecular weight (Mw) of 2,000 to 10,000 g/mol; a crosslinking agent; a polymerization initiator; and a solvent, and a hydrogel formed from the composition.

## Description

### [Technical Field]

The present invention relates to a hydrogel composition and a hydrogel formed by using the same.

### [Background Art]

A hydrogel is a hydrophilic polymer material characterized by forming a network structure in which polymer chains are threedimensionally crosslinked, and capable of absorbing and retaining a large amount of moisture within the structure. Due to this characteristic, hydrogels have mechanical properties similar to those of soft tissues and thus have excellent biocompatibility, and are widely utilized in various fields such as medical patches, tissue engineering scaffolds, drug delivery systems, wound protective materials, cosmetics, and biosensors.

However, a certain amount of moisture is always present on the surface of a hydrogel, and this manifests as a physical phenomenon referred to as 'stickiness' or 'tackiness' upon contact with an external surface during use. Such stickiness originates from inherent moisture retention capacity and hydrophilicity of the hydrogel, which generates excessive adhesion when the hydrogel comes into contact with an external object or skin. As a result, it may cause discomfort or irritation to a user's skin, and contaminants such as dust, fibers and bacteria may easily adhere thereto, causing hygiene-related issues.

In addition, a hydrogel with strong tackiness may cause pain or skin damage upon detachment after use. Particularly, in the case of products requiring repeated attachment and detachment (for example, medical patches, reusable sensor adhesives, and the like), such tackiness may increase user discomfort and may also adversely affect durability of the product. In medical environments, such stickiness may cause interference between devices or lead to patient dissatisfaction due to a foreign body sensation, and from an industrial perspective, it causes problems in terms of consistency in product quality as well as ease of storage and handling.

In order to solve such problems, various approaches to adhesion control have been proposed. For example, attempts have been made to minimize physical contact by forming a thin film on the surface of a hydrogel or attaching a release film thereto, or by controlling the surface composition of a hydrogel to adjust hydrophilicity or hydrophobicity. In addition, studies have been conducted to influence surface properties by controlling internal network density or crosslinking density of a hydrogel.

However, such existing technologies have limitations in various aspects such as practicality, ease of manufacturing, cost, and durability. For example, technologies requiring separate surface processing or complex multi-step manufacturing processes are not suitable for mass production, and often provide only temporary effects. In addition, measures taken to suppress tackiness frequently have negative impacts on the inherent functions of a hydrogel (for example: moisture retention capacity, mechanical elasticity, transparency or the like).

Accordingly, there is still a need for the development of a technology capable of effectively and continuously controlling the surface property of stickiness while maintaining the inherent functions and structural properties of a hydrogel.

### [Prior Art Documents]

### [Patent Documents]

(Patent Document 1) Korean Laid-open Patent Publication No. 10-2018-0008076 (2018.01.24)
(Patent Document 2) Korean Laid-open Patent Publication No. 10-2017-0056423 (2017.05.23)
(Patent Document 3) US Patent No. 5143071 (1992.09.01)

### [Invention]

### [Technical Problem]

It is an object of the present invention to solve the above problems to provide a hydrogel composition including polyethylene glycol (PEG) having a weight average molecular weight (Mw) of 2,000 g/mol to 10,000 g/mol in a specific amount, thereby being capable of effectively controlling surface stickiness while maintaining physical properties and biocompatibility of the hydrogel composition.

It is another object of the present invention to provide a hydrogel having controlled stickiness formed from the composition.

### [Technical Solution]

One embodiment of the present invention provides a hydrogel composition comprising: an acrylic compound including acrylamide, methacrylamide, acrylic acid, or a combination thereof; polyethylene glycol having a weight average molecular weight (Mw) of 2,000 g/mol to 10,000 g/mol; a crosslinking agent; a polymerization initiator; and a solvent.

The acrylic compound may be included in an amount of 10 to 35 parts by weight based on 100 parts by weight of the solvent.

The polyethylene glycol may be included in an amount of 1 to 5 parts by weight based on 100 parts by weight of the solvent.

The polyethylene glycol may have a weight average molecular weight (Mw) of 4,000 to 8,000 g/mol.

The polyethylene glycol may not include a functional group capable of participating in a crosslinking reaction at both ends of the molecule.

The crosslinking agent may be at least one selected from the group consisting of N,N'-methylenebisacrylamide (MBA), trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin, and ethylene carbonate.

The polymerization initiator may be at least one selected from the group consisting of potassium persulfate (KPS), peroxides, azo compounds, azobisisobutyronitrile (AIBN), persulfates, ammonium persulfate (APS), sodium persulfate, hydrogen peroxide, benzoyl peroxide, and ceric ammonium nitrate (CAN).

The solvent may include water, glycerol, or a combination thereof.

When the solvent includes both water and glycerol, the water and the glycerol may be included in a weight ratio of 9:1 to 4:6.

The composition further comprises a natural hydrophilic polymer, and the natural hydrophilic polymer may include agarose, alginate, sodium carboxymethyl cellulose (CMC-Na), or a combination thereof.

Another embodiment of the present invention provides a hydrogel formed from the composition.

### [Advantageous Effects]

According to the present invention, by using an acrylic monomer including acrylamide, methacrylamide, acrylic acid or a combination thereof in the composition, it is possible to secure shape retention and water holding capacity of a hydrogel, so that the hydrogel can be applied without performance deterioration even when used for a long period of time. In addition, by selecting polyethylene glycol (PEG) to have a weight average molecular weight (Mw) in the range of 2,000 g/mol to 10,000 g/mol and including the polyethylene glycol in the composition in a specific amount, the polyethylene glycol is effectively dispersed within the gel structure of the hydrogel, significantly reducing excessive stickiness on the gel surface. In addition, by optimizing the molecular weight and the content of the PEG, it is possible to reduce tackiness and improve wearing comfort while maintaining mechanical properties (for example: elongation, elasticity, durability) of the hydrogel, and stickiness can be controlled without a separate anti-tackiness treatment, thereby simplifying the manufacturing process and improving productivity. Through a combination with a crosslinking agent and a polymerization initiator, the network structure of the gel can be stabilized to obtain a hydrogel having a desired level of flexibility and moisture permeability. The hydrogel composition according to the present invention can be applied to various application fields such as skin-adhesive patches, medical dressings, cosmetic mask packs and biosensor adhesives, and thus is expected to have high industrial applicability.

### [Description of Drawings]

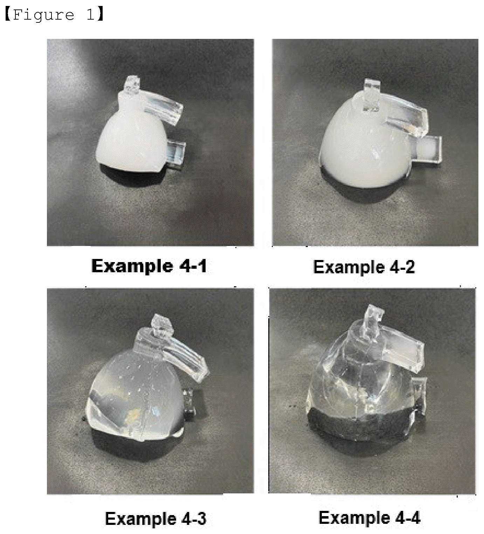

FIG. 1 illustrates an image of a hydrogel according to one embodiment of the present invention.

### [Best Mode]

Hereinafter, embodiments of the present invention will be described in detail. Before this, terms or words used in this specification and the claims are not to be interpreted limitedly to common or dictionary meanings, and need to be interpreted as meanings and concepts corresponding to technical ideas of the present invention based on a principle in which the inventors may suitably define concepts of the terms in order to describe their invention in the best possible way. Accordingly, constitutions described in the embodiments described in this specification are just most preferred one embodiment of the present invention and do not represent all of the technical ideas of the present invention, and therefore, it needs to be understood that there may be various equivalents and modified examples that may replace these at the time of filing the present application.

Throughout this specification, when a part is described as "comprising" a component, this means that, unless particularly stated otherwise, it does not exclude other components but may further include other components.

In addition, description of specifying components by limiting or adding components may be applied to all inventions unless there is a particular restriction, and is not limited to a specific invention.

In addition, throughout the description of invention and the claims of the present application, singular forms include plural forms as well unless otherwise stated.

In addition, throughout the description of invention and the claims of the present application, "or" includes "and" unless otherwise stated. Therefore, "including A or B" means all three cases of including A, including B, or including A and B.

In addition, all numerical ranges include both the endpoint values and all intermediate values therebetween, unless explicitly stated to exclude them.

Hereinafter, a hydrogel composition according to one embodiment of the present invention and a hydrogel formed therethrough will be described.

### Hydrogel Composition

A hydrogel composition according to one embodiment of the present invention includes: an acrylic compound including acrylamide, methacrylamide, acrylic acid, or a combination thereof; polyethylene glycol having a weight average molecular weight (Mw) of 2,000 to 10,000 g/mol; a crosslinking agent; a polymerization initiator; and a solvent.

Hereinafter, each component of the hydrogel composition of the present invention will be described more specifically.

### (1) Acrylic Compound

The acrylic compound included in the hydrogel composition according to the present invention has a hydrophilic functional group, and thus may exhibit strong hydration ability and high absorbency when the composition is cured to form a hydrogel. In addition, since the acrylic compound forms a dense three-dimensional network structure through a crosslinking reaction, it not only enables formation of a hydrogel having excellent tensile strength, elongation and elasticity, but also allows copolymerization with other functional monomers, thereby providing flexibility in designing a hydrogel with tailored properties.

In one embodiment of the present invention, the acrylic compound satisfying such properties may be selected from acrylamide, methacrylamide, acrylic acid, or a combination thereof.

In addition, the composition according to the present invention may further include a natural hydrophilic polymer in addition to the acrylic compound, and the natural hydrophilic polymer may include agarose, alginate, sodium carboxymethyl cellulose (CMC-Na) or a combination thereof. The acrylic compound and the natural hydrophilic polymer may also form a double network through a curing process of the composition, and physical properties such as tensile strength and elongation of a hydrogel may be further strengthened therethrough. Herein, when the natural hydrophilic polymer is sodium carboxymethyl cellulose (CMC-Na), the viscosity may be 400 to 800 cP. When the viscosity is 400 cP or less, the crosslinked structure may not be formed between the acrylic compound and the CMC-Na, and when the viscosity exceeds 800 cP, there is a risk that properties of the formed hydrogel may change, and therefore, it is preferred to use CMC-Na having a viscosity in the above range.

In one embodiment of the present invention, the acrylic compound may be included in an amount of 10 to 35 parts by weight based on 100 parts by weight of the solvent included in the composition, and for example, may be included in an amount of 10 parts by weight or more, 11 parts by weight or more, 12 parts by weight or more, 13 parts by weight or more, 14 parts by weight or more, 15 parts by weight or more, 16 parts by weight or more, 17 parts by weight or more, 18 parts by weight or more, 19 parts by weight or more, 20 parts by weight or more, 21 parts by weight or more or 22 parts by weight or more, and may be 35 parts by weight or less, 34 parts by weight or less, 33 parts by weight or less, 32 parts by weight or less, 31 parts by weight or less, 30 parts by weight or less, 29 parts by weight or less, 28 parts by weight or less, 27 parts by weight or less, 26 parts by weight or less, 25 parts by weight or less, 24 parts by weight or less or 23 parts by weight or less.

When the content of the acrylic compound satisfies the above range, deterioration in hydrogel properties may be prevented, and a phenomenon in which the composition solution boils during the curing process of the composition may be prevented.

In addition, in one embodiment of the present invention, when the composition further includes a natural hydrophilic polymer in addition to the acrylic compound, the natural hydrophilic polymer may be included in an amount of 0.1 to 5 parts by weight based on 100 parts by weight of the solvent included in the composition, and for example, may be included in an amount of 0.1 parts by weight or more, 0.2 parts by weight or more, 0.3 parts by weight or more, 0.4 parts by weight or more, 0.5 parts by weight or more, 0.6 parts by weight or more, 0.7 parts by weight or more, 0.8 parts by weight or more, 0.9 parts by weight or more, 1.0 parts by weight or more, 1.1 parts by weight or more, 1.2 parts by weight or more, 1.3 parts by weight or more, 1.4 parts by weight or more, 1.5 parts by weight or more, 1.6 parts by weight or more, 1.7 parts by weight or more, 1.8 parts by weight or more, 1.9 parts by weight or more, 2.0 parts by weight or more, 2.1 parts by weight or more, 2.2 parts by weight or more, 2.3 parts by weight or more, 2.4 parts by weight or more or 2.5 parts by weight or more, and may be 5.0 parts by weight or less, 4.9 parts by weight or less, 4.8 parts by weight or less, 4.7 parts by weight or less, 4.6 parts by weight or less, 4.5 parts by weight or less, 4.4 parts by weight or less, 4.3 parts by weight or less, 4.2 parts by weight or less, 4.1 parts by weight or less, 4.0 parts by weight or less, 3.9 parts by weight or less, 3.8 parts by weight or less, 3.7 parts by weight or less, 3.6 parts by weight or less, 3.5 parts by weight or less, 3.4 parts by weight or less, 3.3 parts by weight or less, 3.2 parts by weight or less, 3.1 parts by weight or less, 3.0 parts by weight or less, 2.9 parts by weight or less, 2.8 parts by weight or less, 2.7 parts by weight or less or 2.6 parts by weight or less.

When the content of the natural hydrophilic polymer satisfies the above range, deterioration in hydrogel properties may be prevented, and problems occurring during the curing process due to an excessive increase in the viscosity of the composition may be prevented.

### (2) Polyethylene Glycol (PEG)

A hydrogel formed using monomers such as acrylamide or acrylic acid may cause stickiness on the hydrogel surface by including a functional group capable of inducing hydrogen bonding, such as a hydroxyl group (-OH) or an amide group (-CONH₂), in the molecule, and this may lead to problems such as unnecessary stickiness in applications using the hydrogel, and hygiene issues caused by contact with dust in the air.

In the present invention, by including polyethylene glycol having a specific weight average molecular weight in a specific amount in the hydrogel composition including the acrylic compound, inherent functions and structural properties of a hydrogel may be maintained while effectively and continuously controlling the surface property of stickiness of the hydrogel.

Various approaches previously proposed, for example, a method of forming a thin film on the surface of a hydrogel to control stickiness of the hydrogel or attaching a release film thereto to minimize physical contact, a method of controlling the surface composition of a hydrogel to adjust hydrophilicity or hydrophobicity, a method of adjusting internal network density or crosslinking density of a hydrogel to influence surface properties, or the like, require separate surface processing or complex multi-step manufacturing processes, and thus are not suitable for mass production. Such approaches have limits in that they often provide only temporary effects, and measures taken to suppress tackiness frequently have negative impacts on the inherent functions of a hydrogel (for example: moisture retention capacity, mechanical elasticity, transparency or the like). In contrast, the present invention has technical effects in that surface stickiness of a hydrogel may be controlled simply by adding polyethylene glycol having a specific weight average molecular weight in a specific amount into the composition during the manufacturing process.

To this end, in the present invention, the hydrogel composition including the acrylic compound further includes polyethylene glycol having a weight average molecular weight (Mw) of 2,000 to 10,000 g/mol.

The polyethylene glycol induces hydrogen bonding between hydroxyl groups (-OH) present in the molecule and hydroxyl groups (-OH), amide groups (-CONH₂) or the like present in the molecule of the hydrogel formed by the acrylic compound, thereby allowing the functional groups such as the hydroxyl groups (-OH) or the amide groups (-CONH₂) to be positioned inside the hydrogel rather than on the surface of the hydrogel, and thus controlling surface stickiness of the hydrogel.

Herein, the polyethylene glycol according to the present invention may not include a functional group capable of participating in a crosslinking reaction at both ends of the molecule, such as poly(ethylene glycol) diacrylate (PEGDA) or poly(ethylene glycol) dimethacrylate (PEGDMA).

When polyethylene glycol including a functional group capable of participating in a crosslinking reaction at both ends of the molecule, such as polyethylene glycol diacrylate or polyethylene glycol dimethacrylate, is used to form a hydrogel in order to control surface stickiness of the hydrogel formed from the acrylic compound, the polyethylene glycol forms crosslinking bonds with the acrylic compound in the composition and is included in the network of the hydrogel, thereby excessively increasing crosslinking density, and thus causing the hydrogel to become rigid or rather induce sticky properties.

However, when polyethylene glycol that does not include a functional group capable of participating in a crosslinking reaction at both ends of the molecule is included in the composition as in the hydrogel composition according to the present invention, the polyethylene glycol does not directly participate in the hydrogel network, lowering overall crosslinking density of the gel, and as a result, stickiness is reduced, and an advantage of providing soft and flexible feel may be achieved.

In one embodiment of the present invention, the polyethylene glycol preferably has a weight average molecular weight (Mw) of 2,000 to 10,000 g/mol in order to increase the effect of improving stickiness and prevent deterioration in the hydrogel properties. For example, the polyethylene glycol preferably may have a weight average molecular weight (Mw) of 2,000 g/mol or more, 2,100 g/mol or more, 2,200 g/mol or more, 2,300 g/mol or more, 2,400 g/mol or more, 2,500 g/mol or more, 2,600 g/mol or more, 2,700 g/mol or more, 2,800 g/mol or more, 2,900 g/mol or more, 3,000 g/mol or more, 3,100 g/mol or more, 3,200 g/mol or more, 3,300 g/mol or more, 3,400 g/mol or more, 3,500 g/mol or more, 3,600 g/mol or more, 3,700 g/mol or more, 3,800 g/mol or more, 3,900 g/mol or more, 4,000 g/mol or more, 4,100 g/mol or more, 4,200 g/mol or more, 4,300 g/mol or more, 4,400 g/mol or more, 4,500 g/mol or more, 4,600 g/mol or more, 4,700 g/mol or more, 4,800 g/mol or more, 4,900 g/mol or more, 5,000 g/mol or more, 5,100 g/mol or more, 5,200 g/mol or more, 5,300 g/mol or more, 5,400 g/mol or more, 5,500 g/mol or more, 5,600 g/mol or more, 5,700 g/mol or more, 5,800 g/mol or more, 5,900 g/mol or more or 6,000 g/mol or more, and may be 10,000 g/mol or less, 9,900 g/mol or less, 9,800 g/mol or less, 9,700 g/mol or less, 9,600 g/mol or less, 9,500 g/mol or less, 9,400 g/mol or less, 9,300 g/mol or less, 9,200 g/mol or less, 9,100 g/mol or less, 9,000 g/mol or less, 8,900 g/mol or less, 8,800 g/mol or less, 8,700 g/mol or less, 8,600 g/mol or less, 8,500 g/mol or less, 8,400 g/mol or less, 8,300 g/mol or less, 8,200 g/mol or less, 8,100 g/mol or less, 8,000 g/mol or less, 7,900 g/mol or less, 7,800 g/mol or less, 7,700 g/mol or less, 7,600 g/mol or less, 7,500 g/mol or less, 7,400 g/mol or less, 7,300 g/mol or less, 7,200 g/mol or less, 7,100 g/mol or less, 7,000 g/mol or less, 6,900 g/mol or less, 6,800 g/mol or less, 6,700 g/mol or less, 6,600 g/mol or less, 6,500 g/mol or less, 6,400 g/mol or less, 6,300 g/mol or less, 6,200 g/mol or less or 6,100 g/mol or less, and more preferably 4,000 to 8,000 g/mol.

Surface stickiness of a hydrogel is influenced by physical properties, the degree of hydration, surface energy, intermolecular interactions or the like. Since polyethylene glycol is hydrophilic and its physical properties vary depending on the weight average molecular weight, it also affects surface properties of the hydrogel.

When the polyethylene glycol included in the hydrogel composition according to one embodiment of the present invention has a weight average molecular weight of 2,000 g/mol or less, the polyethylene glycol does not effectively remain on the surface of the hydrogel and is likely to diffuse into the hydrogel or be eluted by moisture, and thus the effect of reducing surface stickiness of the hydrogel tends to be lost in a short period of time. In addition, when the polyethylene glycol has a weight average molecular weight exceeding 10,000 g/mol, viscosity is relatively high and fluidity is low, causing a problem in that it is difficult to uniformly disperse the polyethylene glycol in the hydrogel composition. Furthermore, since high-molecular-weight polyethylene glycol shows slow diffusion and rearrangement at the surface, there is a limit in rapidly modifying surface properties of the hydrogel, and as a result, the effect of reducing stickiness of the hydrogel may not be sufficiently exhibited, and interactions with the crosslinked structure of the hydrogel may be limited as well, such that the polyethylene glycol is likely to exist as an inactive component in the hydrogel matrix, which is inefficient in terms of providing a stickiness-reducing effect. Accordingly, polyethylene glycol having a weight average molecular weight in the above range is preferably used.

In one embodiment of the present invention, the polyethylene glycol may be included in an amount of 1 to 5 parts by weight based on 100 parts by weight of the solvent included in the composition, and for example, may be included in an amount of 1.0 part by weight or more, 1.1 parts by weight or more, 1.2 parts by weight or more, 1.3 parts by weight or more, 1.4 parts by weight or more, 1.5 parts by weight or more, 1.6 parts by weight or more, 1.7 parts by weight or more, 1.8 parts by weight or more, 1.9 parts by weight or more, 2.0 parts by weight or more, 2.1 parts by weight or more, 2.2 parts by weight or more, 2.3 parts by weight or more, 2.4 parts by weight or more, 2.5 parts by weight or more, 2.6 parts by weight or more, 2.7 parts by weight or more, 2.8 parts by weight or more, 2.9 parts by weight or more or 3.0 parts by weight or more, and may be 5.0 parts by weight or less, 4.9 parts by weight or less, 4.8 parts by weight or less, 4.7 parts by weight or less, 4.6 parts by weight or less, 4.5 parts by weight or less, 4.4 parts by weight or less, 4.3 parts by weight or less, 4.2 parts by weight or less, 4.1 parts by weight or less, 4.0 parts by weight or less, 3.9 parts by weight or less, 3.8 parts by weight or less, 3.7 parts by weight or less, 3.6 parts by weight or less, 3.5 parts by weight or less, 3.4 parts by weight or less, 3.3 parts by weight or less, 3.2 parts by weight or less or 3.1 parts by weight or less.

When the content of the polyethylene glycol satisfies the above range, the effect of improving surface stickiness of the hydrogel is enhanced, and a phenomenon in which an excessive amount of polyethylene glycol is eluted and transferred onto the surface of the hydrogel may be prevented.

### (3) Crosslinking Agent and Initiator

The crosslinking agent included in the hydrogel composition of the present invention may perform a role of further improving physical, mechanical properties and chemical stability of a hydrogel by crosslinking the acrylic compounds, or the acrylic compound and the natural hydrophilic polymer.

Accordingly, any type of the crosslinking agent may be used as long as it is a material capable of crosslinking the acrylic compounds, or the acrylic compound and the natural hydrophilic polymer in the composition of the present invention, and for example, at least one selected from the group consisting of N,N'-methylenebisacrylamide (MBA), trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin, and ethylene carbonate may be used.

The crosslinking agent may be included in an amount of 0.01 to 0.2 parts by weight based on 100 parts by weight of the solvent included in the composition according to the present invention, and for example, may be included in an amount of 0.01 to 0.1 parts by weight, preferably 0.02 to 0.05 parts by weight. When the content of the crosslinking agent is less than the above range, crosslinking sufficient to impart mechanical strength and chemical stability of a hydrogel may not be achieved, and when the content exceeds the above range, excessive crosslinking may occur, thereby posing a risk of deterioration in properties such as tensile properties.

The polymerization initiator is a material used to initiate curing of the composition, specifically, a chain reaction of materials included in the composition, and may specifically refer to a thermal curing initiator, which is a material readily generating radicals using a heat source or a catalyst.

Any type of the polymerization initiator may be used as long as it is a material capable of initiating a curing reaction by a heat source or a catalyst, and for example, at least one selected from the group consisting of potassium persulfate (KPS), peroxides, azo compounds, azobisisobutyronitrile (AIBN), persulfates, ammonium persulfate (APS), sodium persulfate, hydrogen peroxide, and benzoyl peroxide may be used.

Herein, a polymerization catalyst may be used together with the polymerization initiator in order to promote the generation of free radicals or to increase a rate of the initiation reaction, and for example, N,N,N',N'-tetramethylethylenediamine (TEMED), diethylenetriamine (DETA), ethylenediamine (EDA), N,N-dimethylethylenediamine (DMEDA), trimethylamine (TMA), sodium bisulfite (NaHSO₃), sodium thiosulfate (Na₂S₂O₃), ferrous sulfate (FeSO₄), or ascorbic acid (vitamin C) may be used therewith. The polymerization catalyst may be included in an amount of 0.01 to 0.4 parts by weight based on 100 parts by weight of the solvent included in the composition, and it is preferred not to use an excessive amount in order to prevent hydrogel properties from deteriorating through a rapid curing reaction.

The polymerization initiator may be included in an amount of 0.01 to 0.5 parts by weight based on 100 parts by weight of the solvent included in the composition according to the present invention, and for example, may be included in an amount of 0.1 to 0.5 parts by weight, preferably 0.1 to 0.4 parts by weight. When the content of the initiator is less than the above range, the curing process of the composition according to the present invention may not proceed smoothly or the curing time may be excessively delayed. When the content of the initiator exceeds the above-mentioned range, a rapid radical reaction may proceed, thereby causing side reactions or having unintended effects on other components of the composition according to the present invention, and an excessive amount of residual components may remain after curing.

The hydrogel composition according to the present invention may further include additives such as a surfactant, a plasticizer, a stabilizer, a defoaming agent or a diluent in addition to the crosslinking agent and the polymerization initiator. The additives may be included in a minimum amount necessary for the above-mentioned action to occur for economic reasons, and may be preferably included in an amount of 0.01 to 0.4 parts by weight based on 100 parts by weight of the solvent included in the hydrogel composition.

### (4) Solvent

As the solvent capable of favorably dissolving each component such as the acrylic compound, the polyethylene glycol, the crosslinking agent and the polymerization initiator included in the hydrogel composition of the present invention, a solvent such as water may be used.

In addition, as the polyethylene glycol is additionally included to control surface stickiness in preparing a hydrogel using the acrylic compound, transparency of a hydrogel is reduced due to the phenomenon of phase separation during the curing process of the composition, and in order to address this issue, glycerol may be included in addition to water as the solvent of the composition. Herein, when water and glycerol are both included as the solvent included in the composition, they may be included in a weight ratio of 9:1 to 4:6, and for example, water may be included in an amount of 90 parts by weight or less, 85 parts by weight or less, 80 parts by weight or less, 75 parts by weight or less, 70 parts by weight or less, 65 parts by weight or less, 60 parts by weight or less, 55 parts by weight or less, 50 parts by weight or less, 45 parts by weight or less, or 40 parts by weight or less based on the total 100 parts by weight of water and glycerol. In order to improve transparency of a hydrogel and prevent property deterioration, the glycerol content is preferably adjusted in the above range when including both water and glyceryl as the solvent of the composition.

### Hydrogel

Another embodiment of the present invention provides a hydrogel formed from the composition. Specific components included in the hydrogel composition are the same as those described above, and therefore, detailed descriptions thereof will be omitted.

The hydrogel according to the present invention may be prepared through the following exemplary process using the hydrogel composition.

First, a composition including an acrylic compound including acrylamide, methacrylamide, acrylic acid or a combination thereof, polyethylene glycol having a weight average molecular weight (Mw) of 2,000 to 10,000 g/mol, a crosslinking agent, a polymerization initiator, and, when necessary, a polymerization accelerator was mixed with water, a solvent, to prepare a reaction composition.

The reaction composition is heated to a certain temperature (for example: about 40 to 80°C) to induce thermal initiation polymerization, and is maintained for a certain period of time to be cured into a hydrogel. The curing time and temperature may be adjusted depending on the type and ratio of the composition used.

The hydrogel according to one embodiment of the present invention is characterized in that surface stickiness is significantly suppressed even after curing by including an acrylic compound and polyethylene glycol (PEG) having a weight average molecular weight in a specific range, and due to such properties, the hydrogel exhibits excellent tactile satisfaction of a user and excellent processability in subsequent processes, thereby being usable in various industrial applications.

In particular, the hydrogel has tackiness or stickiness, which may cause discomfort upon contact with the skin, eliminated, and thus may be effectively utilized in skin-applied products such as cosmetic sheet masks, skin-adhesive cooling pads, wet bandages and dressings, so the hydrogel adheres closely to the skin while causing minimal irritation upon removal, and may improve the inherent adhesionrelated drawbacks of hydrogels. In addition, due to its excellent viscoelasticity and moisture retention capacity, the hydrogel is also applicable to medical and bio-healthcare products such as drug delivery systems, transdermal drug patches, medical gel pads, orthopedic splint materials, and gel dressings for wound treatment. Moreover, the hydrogel of the present invention exhibits a balance between transparency and mechanical stability, and has properties in which the surface is not dry while being non-sticky, and therefore, may also be usefully employed as industrial materials such as hydrogel pads for protecting electronic devices, functional cushioning materials, humidity control materials, and functional adhesive sheets.

Accordingly, the hydrogel of the present invention is broadly applicable to various consumer and industrial products, and in particular, may be utilized as a highly functional hydrogel composition suitable for applications aiming to maintain the inherent functionality of a hydrogel while minimizing surface stickiness.

Hereinafter, specific examples of the present invention will be provided. However, the examples described below are only to specifically illustrate or explain the present invention, and the present invention is not limited thereby. In addition, description not provided herein may be sufficiently inferred technically by those skilled in the art, and therefore, the description is not included.

### Example 1-1

(1) At room temperature (about 25°C), 100 g of distilled water was placed in a 250 mL beaker, and while being stirred using a magnetic stirrer, 18 g of acrylamide and 0.0256 g of N,N'-methylenebisacrylamide (MBA) as a crosslinking agent were sequentially added thereto and completely dissolved. Subsequently, 1.18 g of polyethylene glycol (PEG 6000) having a weight average molecular weight of about 6,000 g/mol was added thereto, and then uniformly dissolved through stirring to prepare a mixture solution.
(2) To the mixture solution, 0.1836 g of ammonium persulfate (APS) was added as a polymerization initiator, then 20 µl of N,N,N',N'-tetramethylethylenediamine (TEMED), which is a polymerization accelerator, was added thereto, and then the result was further stirred for about 30 seconds using a magnetic stirrer to prepare a reaction composition.
(3) The thoroughly mixed composition was dispensed into a silicone mold, and then cured for about 10 minutes at room temperature. After the curing was completed, the cured product was demolded to obtain a hydrogel sample having viscoelasticity and reduced surface stickiness.

### Example 1-2

(1) At room temperature (about 25°C), 100 g of distilled water was placed in a 250 mL beaker, and while being stirred using a magnetic stirrer, 18 g of acrylamide and 0.0256 g of N,N'-methylenebisacrylamide (MBA) as a crosslinking agent were sequentially added thereto and completely dissolved. Subsequently, 0.1 g of agarose was added thereto, and then completely dissolved while being heated to 80°C. Subsequently, 1.18 g of polyethylene glycol (PEG 6000) having a weight average molecular weight of about 6,000 g/mol was added thereto, then uniformly dissolved through stirring, and the temperature of the solution was lowered to 40°C to prepare a mixture solution.
(2) To the mixture solution, 0.1836 g of ammonium persulfate (APS) was added as a polymerization initiator, then 20 µl of N,N,N',N'-tetramethylethylenediamine (TEMED), which is a polymerization accelerator, was added thereto, and then the result was further stirred for about 30 seconds using a magnetic stirrer to prepare a reaction composition.
(3) The thoroughly mixed composition was dispensed into a silicone mold, and then cured for about 10 minutes at room temperature. After the curing was completed, the cured product was demolded to obtain a hydrogel sample having viscoelasticity and reduced surface stickiness.

### Example 1-3

A hydrogel sample was obtained in the same manner as in Example 1-2, except that 0.5 g of agarose was introduced.

### Example 1-4

(1) At room temperature (about 25°C), 100 g of distilled water was placed in a 250 mL beaker, and while being stirred using a magnetic stirrer, 18 g of acrylamide and 0.0256 g of N,N'-methylenebisacrylamide (MBA) as a crosslinking agent were sequentially added thereto and completely dissolved. Subsequently, 0.3 g of alginate was added thereto, then 1.2 g of PEG 6000 was further added, and the mixture was rapidly stirred to ensure that both alginate and PEG were completely dissolved, and as a result, a mixture solution was prepared.
(2) To the mixture solution, 0.1836 g of ammonium persulfate (APS) was added as a polymerization initiator, then 20 µl of N,N,N',N'-tetramethylethylenediamine (TEMED), which is a polymerization accelerator, was added thereto, and then the result was further stirred for about 30 seconds using a magnetic stirrer to prepare a reaction composition.
(3) The thoroughly mixed composition was dispensed into a silicone mold, and then cured for about 10 minutes at room temperature. After the curing was completed, the cured product was demolded to obtain a hydrogel sample having viscoelasticity and reduced surface stickiness.

### Example 1-5

A hydrogel sample was obtained in the same manner as in Example 1-1, except that 30 g of acrylamide, 0.048 g of N,N'-methylenebisacrylamide (MBA) as the crosslinking agent, 0.3672 g of ammonium persulfate (APS) as the polymerization initiator, and 2.6 g of PEG 6000 were introduced.

### Example 1-6

A hydrogel sample was obtained in the same manner as in Example 1-2, except that 30 g of acrylamide, 0.048 g of N,N'-methylenebisacrylamide (MBA) as the crosslinking agent, 0.3672 g of ammonium persulfate (APS) as the polymerization initiator, and 2.6 g of PEG 6000 were introduced.

### Example 1-7

A hydrogel sample was obtained in the same manner as in Example 1-6, except that 0.5 g of agarose was introduced.

### Example 1-8

A hydrogel sample was obtained in the same manner as in Example 1-4, except that 30 g of acrylamide, 0.048 g of N,N'-methylenebisacrylamide (MBA) as the crosslinking agent, 0.3672 g of ammonium persulfate (APS) as the polymerization initiator, and 2.6 g of PEG 6000 were introduced.

### Comparative Example 1-1

A hydrogel sample was obtained in the same manner as in Example 1-1, except that polyethylene glycol (PEG 6000) having a weight average molecular weight of about 6,000 g/mol was not added.

### Comparative Example 1-2

A hydrogel sample was obtained in the same manner as in Example 1-1, except that 0.3 g of polyethylene glycol (PEG 6000) having a weight average molecular weight of about 6,000 g/mol was introduced.

### Comparative Example 1-3

A hydrogel sample was obtained in the same manner as in Example 1-1, except that 1.18 g of polyethylene glycol (PEG 200) having a weight average molecular weight of about 200 g/mol was introduced instead of polyethylene glycol (PEG 6000) having a weight average molecular weight of about 6,000 g/mol.

### Comparative Example 1-4

A hydrogel sample was obtained in the same manner as in Example 1-2, except that polyethylene glycol (PEG 6000) having a weight average molecular weight of about 6,000 g/mol was not added.

### Comparative Example 1-5

A hydrogel sample was obtained in the same manner as in Example 1-3, except that polyethylene glycol (PEG 6000) having a weight average molecular weight of about 6,000 g/mol was not added.

### Comparative Example 1-6

A hydrogel sample was obtained in the same manner as in Example 1-4, except that polyethylene glycol (PEG 6000) having a weight average molecular weight of about 6,000 g/mol was not added.

### Comparative Example 1-7

A hydrogel sample was obtained in the same manner as in Example 1-4, except that 1.2 g of polyethylene glycol (PEG 200) having a weight average molecular weight of about 200 g/mol was introduced instead of polyethylene glycol (PEG 6000) having a weight average molecular weight of about 6,000 g/mol.

### Comparative Example 1-8

A hydrogel sample was obtained in the same manner as in Example 1-5, except that polyethylene glycol (PEG 6000) having a weight average molecular weight of about 6,000 g/mol was not added.

### Comparative Example 1-9

A hydrogel sample was obtained in the same manner as in Example 1-5, except that 0.5 g of polyethylene glycol (PEG 6000) having a weight average molecular weight of about 6,000 g/mol was introduced.

### Comparative Example 1-10

A hydrogel sample was obtained in the same manner as in Example 1-5, except that 2.6 g of polyethylene glycol (PEG 200) having a weight average molecular weight of about 200 g/mol was introduced instead of polyethylene glycol (PEG 6000) having a weight average molecular weight of about 6,000 g/mol.

### Comparative Example 1-11

A hydrogel sample was obtained in the same manner as in Example 1-6, except that polyethylene glycol (PEG 6000) having a weight average molecular weight of about 6,000 g/mol was not added.

### Comparative Example 1-12

A hydrogel sample was obtained in the same manner as in Example 1-7, except that polyethylene glycol (PEG 6000) having a weight average molecular weight of about 6,000 g/mol was not added.

### Comparative Example 1-13

A hydrogel sample was obtained in the same manner as in Example 1-7, except that 2.6 g of polyethylene glycol (PEG 200) having a weight average molecular weight of about 200 g/mol was introduced instead of polyethylene glycol (PEG 6000) having a weight average molecular weight of about 6,000 g/mol.

### Comparative Example 1-14

A hydrogel sample was obtained in the same manner as in Example 1-8, except that polyethylene glycol (PEG 6000) having a weight average molecular weight of about 6,000 g/mol was not added.

### Comparative Example 1-15

A hydrogel sample was obtained in the same manner as in Example 1-8, except that 2.6 g of polyethylene glycol (PEG 200) having a weight average molecular weight of about 200 g/mol was introduced instead of polyethylene glycol (PEG 6000) having a weight average molecular weight of about 6,000 g/mol.

### Example 2-1

(1) At room temperature (about 25°C), 100 g of distilled water was placed in a 250 mL beaker, and while being stirred using a magnetic stirrer, 20 g of acrylic acid and 0.032 g of N,N'-methylenebisacrylamide (MBA) as a crosslinking agent were sequentially added thereto and completely dissolved. Subsequently, 1.2 g of polyethylene glycol (PEG 6000) having a weight average molecular weight of about 6,000 g/mol was added thereto, and then uniformly dissolved through stirring to prepare a mixture solution.
(2) To the mixture solution, 0.2448 g of ammonium persulfate (APS) was added as a polymerization initiator, then 20 µl of N,N,N',N'-tetramethylethylenediamine (TEMED), which is a polymerization accelerator, was added thereto, and then the result was further stirred for about 30 seconds using a magnetic stirrer to prepare a reaction composition.
(3) The thoroughly mixed composition was dispensed into a silicone mold, and then cured for about 10 minutes at room temperature. After the curing was completed, the cured product was demolded to obtain a hydrogel sample having viscoelasticity and reduced surface stickiness.

### Example 2-2

(1) At room temperature (about 25°C), 100 g of distilled water was placed in a 250 mL beaker, and while being stirred using a magnetic stirrer, 20 g of acrylic acid and 0.032 g of N,N'-methylenebisacrylamide (MBA) as a crosslinking agent were sequentially added thereto and completely dissolved. Subsequently, 0.5 g of agarose was added thereto, and then completely dissolved while being heated to 80°C. Subsequently, 1.2 g of polyethylene glycol (PEG 6000) having a weight average molecular weight of about 6,000 g/mol was added thereto, then uniformly dissolved through stirring, and the temperature of the solution was lowered to 40°C to prepare a mixture solution.
(2) To the mixture solution, ammonium persulfate (APS) (0.2448 g) was added as a polymerization initiator, then N,N,N',N'-tetramethylethylenediamine (TEMED) (20 µl), which is a polymerization accelerator, was added thereto, and then the result was further stirred for about 30 seconds using a magnetic stirrer to prepare a reaction composition.
(3) The thoroughly mixed composition was dispensed into a silicone mold, and then cured for about 10 minutes at room temperature. After the curing was completed, the cured product was demolded to obtain a hydrogel sample having viscoelasticity and reduced surface stickiness.

### Example 2-3

(1) At room temperature (about 25°C), 100 g of distilled water was placed in a 250 mL beaker, and while being stirred using a magnetic stirrer, 20 g of acrylic acid and 0.032 g of N,N'-methylenebisacrylamide (MBA) as a crosslinking agent were sequentially added thereto and completely dissolved. Subsequently, 2.25 g of sodium carboxymethyl cellulose (CMC-Na) was added thereto, and then completely dissolved while being heated to 60°C. Subsequently, 1.4 g of polyethylene glycol (PEG 6000) having a weight average molecular weight of about 6,000 g/mol was added thereto, then uniformly dissolved through stirring, and the temperature of the solution was lowered to 40°C to prepare a mixture solution.
(2) To the mixture solution, 0.2448 g of ammonium persulfate (APS) was added as a polymerization initiator, then 20 µl of N,N,N',N'-tetramethylethylenediamine (TEMED), which is a polymerization accelerator, was added thereto, and then the result was further stirred for about 30 seconds using a magnetic stirrer to prepare a reaction composition.
(3) The thoroughly mixed composition was dispensed into a silicone mold, and then cured for about 10 minutes at room temperature. After the curing was completed, the cured product was demolded to obtain a hydrogel sample having viscoelasticity and reduced surface stickiness.

### Comparative Example 2-1

A hydrogel sample was obtained in the same manner as in Example 2-1, except that polyethylene glycol (PEG 6000) having a weight average molecular weight of about 6,000 g/mol was not added.

### Comparative Example 2-2

A hydrogel sample was obtained in the same manner as in Example 2-1, except that 1.2 g of polyethylene glycol (PEG 200) having a weight average molecular weight of about 200 g/mol was added instead of polyethylene glycol (PEG 6000) having a weight average molecular weight of about 6,000 g/mol.

### Comparative Example 2-3

A hydrogel sample was obtained in the same manner as in Example 2-2, except that polyethylene glycol (PEG 6000) having a weight average molecular weight of about 6,000 g/mol was not added.

### Comparative Example 2-4

A hydrogel sample was obtained in the same manner as in Example 2-2, except that 1.2 g of polyethylene glycol (PEG 200) having a weight average molecular weight of about 200 g/mol was added instead of polyethylene glycol (PEG 6000) having a weight average molecular weight of about 6,000 g/mol.

### Comparative Example 2-5

A hydrogel sample was obtained in the same manner as in Example 2-3, except that polyethylene glycol (PEG 6000) having a weight average molecular weight of about 6,000 g/mol was not added.

### Example 3-1

(1) At room temperature (about 25°C), 100 g of distilled water was placed in a 250 mL beaker, and while being stirred using a magnetic stirrer, 15 g of methacrylamide and 0.024 g of N,N'-methylenebisacrylamide (MBA) as a crosslinking agent were sequentially added thereto and completely dissolved. Subsequently, 1.3 g of polyethylene glycol (PEG 6000) having a weight average molecular weight of about 6,000 g/mol was added thereto, and then uniformly dissolved through stirring to prepare a mixture solution.
(2) To the mixture solution, 0.1886 g of ammonium persulfate (APS) was added as a polymerization initiator, then 20 µl of N,N,N',N'-tetramethylethylenediamine (TEMED), which is a polymerization accelerator, was added thereto, and then the result was further stirred for about 30 seconds using a magnetic stirrer to prepare a reaction composition.
(3) The thoroughly mixed composition was dispensed into a silicone mold, and then cured for about 10 minutes at room temperature. After the curing was completed, the cured product was demolded to obtain a hydrogel sample having viscoelasticity and reduced surface stickiness.

### Example 3-2

(1) At room temperature (about 25°C), 100 g of distilled water was placed in a 250 mL beaker, and while being stirred using a magnetic stirrer, 15 g of methacrylamide and 0.024 g of N,N'-methylenebisacrylamide(MBA) as a crosslinking agent were sequentially added thereto and completely dissolved. Subsequently, 0.5 g of agarose was added thereto, and then completely dissolved while being heated to 80°C. Subsequently, 1.0 g of polyethylene glycol (PEG 6000) having a weight average molecular weight of about 6,000 g/mol was added thereto, then uniformly dissolved through stirring, and the temperature of the solution was lowered to 40°C to prepare a mixture solution.
(2) To the mixture solution, 0.1886 g of ammonium persulfate (APS) was added as a polymerization initiator, then 20 µl of N,N,N',N'-tetramethylethylenediamine (TEMED), which is a polymerization accelerator, was added thereto, and then the result was further stirred for about 30 seconds using a magnetic stirrer to prepare a reaction composition.
(3) The thoroughly mixed composition was dispensed into a silicone mold, and then cured for about 10 minutes at room temperature. After the curing was completed, the cured product was demolded to obtain a hydrogel sample having viscoelasticity and reduced surface stickiness.

### Comparative Example 3-1

A hydrogel sample was obtained in the same manner as in Example 3-1, except that polyethylene glycol (PEG 6000) having a weight average molecular weight of about 6,000 g/mol was not added.

### Comparative Example 3-2

A hydrogel sample was obtained in the same manner as in Example 3-2, except that polyethylene glycol (PEG 6000) having a weight average molecular weight of about 6,000 g/mol was not added.

### Example 4-1

(1) At room temperature (about 25°C), 100 g of distilled water was placed in a 250 mL beaker, and while being stirred using a magnetic stirrer, 24 g of acrylamide and 0.0384 g of N,N'-methylenebisacrylamide (MBA) as a crosslinking agent were sequentially added thereto and completely dissolved. Subsequently, 1.89 g of polyethylene glycol (PEG 6000) having a weight average molecular weight of about 6,000 g/mol was added thereto, and then uniformly dissolved through stirring to prepare a mixture solution.
(2) To the mixture solution, 0.2448 g of ammonium persulfate (APS) was added as a polymerization initiator, then 20 µl of N,N,N',N'-tetramethylethylenediamine (TEMED), which is a polymerization accelerator, was added thereto, and then the result was further stirred for about 30 seconds using a magnetic stirrer to prepare a reaction composition.
(3) The thoroughly mixed composition was dispensed into a silicone mold, and then cured for about 10 minutes at room temperature. After the curing was completed, the cured product was demolded to obtain a hydrogel sample having viscoelasticity and reduced surface stickiness.

### Example 4-2

(1) At room temperature (about 25°C), 84.11 g of distilled water and 14.01 g of glycerol were placed in a 250 mL beaker, and while being stirred using a magnetic stirrer, 24 g of acrylamide and 0.0384 g of N,N'-methylenebisacrylamide (MBA) as a crosslinking agent were sequentially added thereto and completely dissolved. Subsequently, 1.89 g of polyethylene glycol (PEG 6000) having a weight average molecular weight of about 6,000 g/mol was added thereto, and then uniformly dissolved through stirring to prepare a mixture solution.
(2) To the mixture solution, 0.2448 g of ammonium persulfate (APS) was added as a polymerization initiator, then 20 µl of N,N,N',N'-tetramethylethylenediamine (TEMED), which is a polymerization accelerator, was added thereto, and then the result was further stirred for about 30 seconds using a magnetic stirrer to prepare a reaction composition.
(3) The thoroughly mixed composition was dispensed into a silicone mold, and then cured for about 10 minutes at room temperature. After the curing was completed, the cured product was demolded to obtain a hydrogel sample having viscoelasticity and reduced surface stickiness.

### Example 4-3

A hydrogel sample was obtained in the same manner as in Example 4-2, except that 66.59 g of distilled water and 31.53 g of glycerol were introduced.

### Example 4-4

A hydrogel sample was obtained in the same manner as in Example 4-2, except that 44.07 g of distilled water and 54.05 g of glycerol were introduced.

Each composition of the hydrogel compositions according to the Examples and the Comparative Examples are as shown in Tables 1 to 4 below.

**[Table 1]**

| Classification | Water (g) | AAm* (g) | Agar** (g) | MBA (g) | APS (g) | TEMED (µl) | PEG 200 (g) | PEG 6000 (g) |
|---|---|---|---|---|---|---|---|---|
| Example 1-1 | 100 | 18 | 0 | 0.0256 | 0.1836 | 20 | 0 | 1.18 |
| Example 1-2 | 100 | 18 | 0.1 | 0.0256 | 0.1836 | 20 | 0 | 1.18 |
| Example 1-3 | 100 | 18 | 0.5 | 0.0256 | 0.1836 | 20 | 0 | 1.18 |
| Example 1-5 | 100 | 30 | 0 | 0.048 | 0.3672 | 20 | 0 | 2.6 |
| Example 1-6 | 100 | 30 | 0.1 | 0.048 | 0.3672 | 20 | 0 | 2.6 |
| Example 1-7 | 100 | 30 | 0.5 | 0.048 | 0.3672 | 20 | 0 | 2.6 |
| Comparative Example 1-1 | 100 | 18 | 0 | 0.0256 | 0.1836 | 20 | 0 | 0 |
| Comparative Example 1-2 | 100 | 18 | 0 | 0.0256 | 0.1836 | 20 | 0 | 0.3 |
| Comparative Example 1-3 | 100 | 18 | 0 | 0.0256 | 0.1836 | 20 | 1.18 | 0 |
| Comparative Example 1-4 | 100 | 18 | 0.1 | 0.0256 | 0.1836 | 20 | 0 | 0 |
| Comparative Example 1-5 | 100 | 18 | 0.5 | 0.0256 | 0.1836 | 20 | 0 | 0 |
| Comparative Example 1-8 | 100 | 30 | 0 | 0.048 | 0.3672 | 20 | 0 | 0 |
| Comparative Example 1-9 | 100 | 30 | 0 | 0.048 | 0.3672 | 20 | 0 | 0.5 |
| Comparative Example 1-10 | 100 | 30 | 0 | 0.048 | 0.3672 | 20 | 2.6 | 0 |
| Comparative Example 1-11 | 100 | 30 | 0.1 | 0.048 | 0.3672 | 20 | 0 | 0 |
| Comparative Example 1-12 | 100 | 30 | 0.5 | 0.048 | 0.3672 | 20 | 0 | 0 |
| Comparative Example 1-13 | 100 | 30 | 0.5 | 0.048 | 0.3672 | 20 | 2.6 | 0 |
| Example 1-4 | 100 | 18 | 0.3 | 0.0256 | 0.1836 | 20 | 0 | 1.2 |
| Example 1-8 | 100 | 30 | 0.5 | 0.048 | 0.3672 | 20 | 0 | 2.6 |
| Comparative Example 1-6 | 100 | 18 | 0.3 | 0.0256 | 0.1836 | 20 | 0 | 0 |
| Comparative Example 1-7 | 100 | 18 | 0.3 | 0.0256 | 0.1836 | 20 | 1.2 | 0 |
| Comparative Example 1-14 | 100 | 30 | 0.5 | 0.048 | 0.3672 | 20 | 0 | 0 |
| Comparative Example 1-15 | 100 | 30 | 0.5 | 0.048 | 0.3672 | 20 | 2.6 | 0 |

| | | | | | | | | |
|---|---|---|---|---|---|---|---|---|
| * AAm: Acrylamide ** Agar: Agarose *** Alg: Alginate | | | | | | | | |

**[Table 2]**

| Classification | Water (g) | Acrylic Acid (g) | Agar* (g) | MBA (g) | APS (g) | TEMED (µl) | PEG 200 (g) | PEG 6000 (g) |
|---|---|---|---|---|---|---|---|---|
| Example 2-1 | 100 | 20 | 0 | 0.032 | 0.2448 | 20 | 0 | 1.2 |
| Example 2-2 | 100 | 20 | 0.5 | 0.032 | 0.2448 | 20 | 0 | 1.2 |
| Comparative Example 2-1 | 100 | 20 | 0 | 0.032 | 0.2448 | 20 | 0 | 0 |
| Comparative Example 2-2 | 100 | 20 | 0 | 0.032 | 0.2448 | 20 | 1.2 | 0 |
| Comparative Example 2-3 | 100 | 20 | 0.5 | 0.032 | 0.2448 | 20 | 0 | 0 |
| Comparative Example 2-4 | 100 | 20 | 0.5 | 0.032 | 0.2448 | 20 | 1.2 | 0 |
| Classification | Water (g) | Acrylic Acid (g) | CMC** (g) | MBA (g) | APS (g) | TEMED (µl) | PEG 200 (g) | PEG 6000 (g) |
| Example 2-3 | 100 | 20 | 2.25 | 0.032 | 0.2448 | 20 | 0 | 1.4 |
| Comparative Example 2-5 | 100 | 20 | 2.25 | 0.032 | 0.2448 | 20 | 0 | 0 |

| | | | | | | | | |
|---|---|---|---|---|---|---|---|---|
| * Agar: Agarose ** CMC: sodium carboxymethyl cellulose (CMC-Na) | | | | | | | | |

**[Table 3]**

| Classification | Water (g) | MAAm* (g) | Agar** (g) | MBA (g) | APS (g) | TEMED (µl) | PEG 200 (g) | PEG 6000 (g) |
|---|---|---|---|---|---|---|---|---|
| Example 3-1 | 100 | 15 | 0 | 0.024 | 0.1886 | 20 | 0 | 1.3 |
| Example 3-2 | 100 | 15 | 0.5 | 0.024 | 0.1886 | 20 | 0 | 1 |
| Comparative Example 3-1 | 100 | 15 | 0 | 0.024 | 0.1886 | 20 | 0 | 0 |
| Comparative Example 3-2 | 100 | 15 | 0.5 | 0.024 | 0.1886 | 20 | 0 | 0 |

| | | | | | | | | |
|---|---|---|---|---|---|---|---|---|
| * MAAm: Methacrylamide ** Agar: Agarose | | | | | | | | |

**[Table 4]**

| Classificat ion | Water (g) | AAm* (g) | Agar** (g) | MBA (g) | APS (g) | TEMED (µl) | PEG 6000 (g) | Glycerol (g) |
|---|---|---|---|---|---|---|---|---|
| Example 4-1 | 100 | 24 | 0.1 | 0.0384 | 0.2448 | 20 | 1.89 | 0 |
| Example 4-2 | 84.11 | 24 | 0.1 | 0.0384 | 0.2448 | 20 | 1.89 | 14.01 |
| Example 4-3 | 66.59 | 24 | 0.1 | 0.0384 | 0.2448 | 20 | 1.89 | 31.53 |
| Example 4-4 | 44.07 | 24 | 0.1 | 0.0384 | 0.2448 | 20 | 1.89 | 54.05 |

| | | | | | | | | |
|---|---|---|---|---|---|---|---|---|
| * AAm: Acrylamide ** Agar: Agarose | | | | | | | | |

### Experimental Example 1: Peel Test

A peel test was performed to evaluate adhesive properties of the hydrogel samples prepared in the Examples and the Comparative Examples. The hydrogel sample was prepared using a mold produced using a 3D printer, and a mechanical property measuring device manufactured by YEONJIN S-Tech Corporation was used to measure peel force.

The test was performed according to the following procedure. First, the hydrogel sample having length of 100 mm, a thickness of 3 mm and a width of 20 mm was uniformly attached to a thin steel plate. After that, the specimen was firmly fixed using a roller grip, and then pulled at a tensile speed of 3 mm/s to measure peel force. The peel measurement interval was set from 15 mm to 20 mm, and the change in force was recorded until the sample was completely peeled off from the steel plate. Among the measured data, maximum force (maximum peel force, N) was used as a key indicator of this experimental example, and the results are shown in the following Table 5.

### Experimental Example 2: Adhesion Test

In order to evaluate tackiness properties of the hydrogels prepared in the Examples 1-4 to 1-8 and Comparative Examples 1-6 to 1-15, an adhesion test was performed. This test was performed by measuring the time required for the hydrogel to naturally detach from a substrate due to gravity after being attached to the substrate under a constant load.

The test was performed according to the following procedure. First, a thin stainless steel plate having a size of 200 mm x 200 mm was prepared as a test substrate. Subsequently, the hydrogel sample having a size of 85 mm x 85 mm and a mass of 60 g was prepared. After uniformly attaching the hydrogel sample to the surface of the test substrate, the substrate was lifted in a vertical direction. Herein, the time required for the hydrogel sample to be completely detached from the steel plate was measured in seconds (s), and adhesion was evaluated therethrough. The results are shown in the following Table 5.

**[Table 5]**

| Classification | Peel Test (N) | Adhesion Test (s) |
|---|---|---|
| Example 1-1 | 0.13±0.01 | - |
| Example 1-2 | 0.11±0.01 | - |
| Example 1-3 | 0.08±0.02 | - |
| Example 1-4 | 0.12±0.01 | 6 |
| Example 1-5 | 0.12±0.02 | 5 |
| Example 1-6 | 0.11±0.03 | 3 |
| Example 1-7 | 0.1±0.01 | 4 |
| Example 1-8 | 0.13±0.02 | 5 |
| Comparative Example 1-1 | 0.24±0.05 | - |
| Comparative Example 1-2 | 0.25±0.02 | - |
| Comparative Example 1-3 | 0.25±0.03 | - |
| Comparative Example 1-4 | 0.23±0.03 | - |
| Comparative Example 1-5 | 0.14±0.02 | - |
| Comparative Example 1-6 | 0.25±0.02 | > 600 |
| Comparative Example 1-7 | 0.23±0.01 | > 600 |
| Comparative Example 1-8 | 0.31±0.07 | > 600 |
| Comparative Example 1-9 | 0.28±0.02 | > 600 |
| Comparative Example 1-10 | 0.29±0.05 | > 600 |
| Comparative Example 1-11 | 0.28±0.06 | > 600 |
| Comparative Example 1-12 | 0.17±0.02 | 318 |
| Comparative Example 1-13 | 0.15±0.01 | 298 |
| Comparative Example 1-14 | 0.27±0.03 | > 600 |
| Comparative Example 1-15 | 0.26±0.01 | > 600 |
| | | |
| Example 2-1 | 0.15±0.01 | - |
| Example 2-2 | 0.09±0.01 | - |
| Example 2-3 | 0.12±0.02 | - |
| Comparative Example 2-1 | 0.29±0.02 | - |
| Comparative Example 2-2 | 0.30±0.01 | - |
| Comparative Example 2-3 | 0.17±0.02 | - |
| Comparative Example 2-4 | 0.18±0.01 | - |
| Comparative Example 2-5 | 0.20±0.01 | - |
| | | |
| Example 3-1 | 0.14±0.01 | - |
| Example 3-2 | 0.08±0.01 | - |
| Comparative Example 3-1 | 0.21±0.03 | - |
| Comparative Example 3-2 | 0.13±0.02 | - |

Referring to Table 5 above, it can be seen that the hydrogel formed from the composition including an acrylic compound such as acrylamide, methacrylamide or acrylic acid, or the composition further including a natural hydrophilic polymer together with the above-described type of acrylic compound has improved surface stickiness by further including polyethylene glycol. In particular, it can be seen that the effect of improving surface stickiness of the hydrogel is insignificant when 2,000 g/mol to 10,000 g/mol of the weight average molecular weight (Mw) of the polyethylene glycol is less than the above range, or when the content of the polyethylene glycol having the weight average molecular weight is low in the composition.

### Experimental Example 3: Evaluation of Hydrogel Transparency

Transparency of the hydrogels prepared in the Examples 4-1 to 4-4 was evaluated.

Referring to FIG. 1, when polyethylene glycol was added to the composition in order to improve surface stickiness of the hydrogel, a phenomenon in which the hydrogel became opaque during the curing process occurred. However, it can be confirmed that such a phenomenon was further improved by adding glycerol in addition to water as a solvent of the composition.

Hereinbefore, preferred examples of the present invention have been described in detail, however, the scope of a right of the present invention is not limited thereto, and various modified and improved forms made by those skilled in the art using the basic concept of the present invention defined in the claims also fall within the scope of a right of the present invention.

## Claims

1. A hydrogel composition, comprising:
an acrylic compound including acrylamide, methacrylamide, acrylic acid, or a combination thereof;
polyethylene glycol having a weight average molecular weight (Mw) of 2,000 to 10,000 g/mol;
a crosslinking agent;
a polymerization initiator; and
a solvent.

2. The hydrogel composition of claim 1, wherein the acrylic compound is included in an amount of 10 to 35 parts by weight based on 100 parts by weight of the solvent.

3. The hydrogel composition of claim 1, wherein the polyethylene glycol is included in an amount of 1 to 5 parts by weight based on 100 parts by weight of the solvent.

4. The hydrogel composition of claim 1, wherein the polyethylene glycol has a weight average molecular weight (Mw) of 4,000 to 8,000 g/mol.

5. The hydrogel composition of claim 1, wherein the polyethylene glycol does not include a functional group capable of participating in a crosslinking reaction at both ends of the molecule.

6. The hydrogel composition of claim 1, wherein the crosslinking agent is at least one selected from the group consisting of N,N'-methylenebisacrylamide (MBA), trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin, and ethylene carbonate.

7. The hydrogel composition of claim 1, wherein the polymerization initiator is at least one selected from the group consisting of potassium persulfate (KPS), peroxides, azo compounds, azobisisobutyronitrile (AIBN), persulfates, ammonium persulfate (APS), sodium persulfate, hydrogen peroxide, and benzoyl peroxide.

8. The hydrogel composition of claim 1, wherein the solvent includes water, glycerol, or a combination thereof.

9. The hydrogel composition of claim 8, wherein, when the solvent includes both water and glycerol, the water and the glycerol are included in a weight ratio of 9:1 to 4:6.

10. The hydrogel composition of claim 1, further comprising a natural hydrophilic polymer,
wherein the natural hydrophilic polymer includes agarose, alginate, sodium carboxymethyl cellulose (CMC-Na), or a combination thereof.

11. A hydrogel formed from the composition of claim 1.
